# EUROPEAN PATENT APPLICATION

(11) **EP 4 831 290 A1**
(43) Date of publication of application: **28.10.2026**
(21) Application number: 24906205.0
(22) Date of filing: 11.12.2024
(51) Int. Cl.: C12N 15/62, C12N 15/867, C12N 5/10, C07K 19/00, A61K 39/00, A61P 35/00, A61P 35/02

(54) **BISPECIFIC CHIMERIC ANTIGEN RECEPTOR TARGETING CD19 AND BCMA**

(30) Priority: 21.12.2023 CN 202311781807
(71) Applicant: Shenzhen Pregene Biopharma Co., Ltd., Shenzhen, Guangdong 518122 (CN)
(72) Inventor: ZHANG, Jishuai, Shenzhen, Guangdong 518122 (CN); LI, Hongjian, Shenzhen, Guangdong 518122 (CN); LI, Yanyang, Shenzhen, Guangdong 518122 (CN); WANG, Meiling, Shenzhen, Guangdong 518122 (CN); SU, Hongchang, Shenzhen, Guangdong 518122 (CN); SUN, Yaowei, Shenzhen, Guangdong 518122 (CN); LU, Qiumei, Shenzhen, Guangdong 518122 (CN); LUO, Yanping, Shenzhen, Guangdong 518122 (CN); ZHANG, Shuren, Shenzhen, Guangdong 518122 (CN); GUO, Mengmeng, Shenzhen, Guangdong 518122 (CN)
(74) Representative: Murgitroyd & Company
(86) International application number: PCT/CN2024/138500
(87) International publication number: WO 2025/130722

(57) **Abstract**

The present invention relates to a bispecific chimeric antigen receptor (CAR) targeting CD19 and BCMA. The CAR comprises an extracellular antigen binding domain, and the extracellular antigen binding domain comprises a first antigen binding domain and a second antigen binding domain, wherein the first antigen binding domain binds to CD19, and the second antigen binding domain binds to BCMA.

## Description

### TECHNICAL FIELD

The present application relates to the field of biomedicine, and in particular to a bispecific chimeric antigen receptor targeting CD19 and BCMA and use thereof.

### BACKGROUND

CD19 is a transmembrane glycoprotein expressed on the surface of B cell lineage cells with a size of 95 KDa. As a member of the immunoglobulin (Ig) superfamily, CD19 is a component element of a signal transduction complex on the surface of B cells, participates in regulating the signal transduction process of B cell receptors, and plays an important role in regulating the activation of B lymphocytes. In addition to pluripotent hematopoietic stem cells and plasma cells, CD19 is expressed at all stages of B lymphocyte differentiation and even during transformation into tumors. CD19 is also expressed on the surface of most B cell lymphomas, mantle cell lymphomas, ALLs, CLLs, and a portion of acute myeloid leukemia cells. CD19 is not expressed in hematopoietic stem cells, and this characteristic reduces the risk of autoimmune diseases and irreversible spinal cord injury during treatment with CD19-targeted drugs. Therefore, CD19 is considered to be a highly promising immunotherapeutic target in treatment of CD19-associated tumors.

B cell maturation antigen (BCMA), also known as CD269, is a member of the tumor necrosis factor superfamily (tumor necrosis factor receptor superfamily member 17, TNFRS17). It consists of 185 amino acid residues, is a type III transmembrane protein, has a cysteine-rich extracellular domain, and has certain conservation. As a cell surface protein expressed only on the B cell lineage, it, together with other 2 TNF receptor superfamily members, B cell activation factor (BAFF) and its receptor (BAFF-R), and transmembrane activator and calcium modulator and cyclophilin ligand (TACI), plays an important role in regulating the proliferation, survival, maturation, and differentiation of B cells into plasma cells. Unlike BAFF-R and TACI, BCMA is mainly expressed on the surface of plasma cells or malignant plasma cells, and its expression can also be detected on the surface of some memory B cells capable of differentiating into malignant plasma cells and plasmacytoid dendritic cells. BCMA is substantially not expressed in naive B cells, pluripotent stem cells, and non-lymphoid tissue. In addition, BCMA is expressed in almost all multiple myeloma (MM) cell lines, and the expression of BCMA in malignant plasma cells is higher than that in normal plasma cells. This evidence makes BCMA a promising ideal target for CAR T cell therapy of MM.

At present, the first CAR-T drug on the market is Kymariah, which targets CD19 and has a significant long-term efficacy in treating patients with refractory/recurrent B cell acute lymphocytic leukemia. Equecabtagene autoleucel injection, a BCMA CAR-T drug, achieved an overall response rate (ORR) of 96%.

Although CD19 CAR-T can effectively target most B cells, it cannot eliminate terminally differentiated plasma cells, whereas BCMA CAR-T can effectively eliminate plasma cells but cannot target naïve B cells, etc. The indications of the two single-target CAR-T cell therapies are limited, and tumor cells are prone to immune escape due to a reduction in target proteins. Therefore, the development of a chimeric antigen receptor capable of simultaneously targeting CD19 and BCMA targets and reducing tumor immune escape caused by reduced expression of the target protein is the key to solving the problems described above.

### SUMMARY

The present application provides a chimeric antigen receptor capable of simultaneously targeting CD19 and BCMA, which can specifically bind to and immunologically recognize two different antigens, CD19 and BCMA, and the chimeric antigen receptor can generate an immune response by binding to either CD19 or BCMA, and can also generate an immune response by simultaneously binding to CD19 and BCMA.

Compared with a chimeric antigen receptor targeting a single antigen, the chimeric antigen receptor provided in the present application can target CD19 and BCMA. Immune cells expressing the chimeric antigen receptor exhibit enhanced cellular activity and a stronger killing effect, and can effectively kill cells that express CD19 and/or BCMA. The CD19 and BCMA targets have an additive or synergistic effect and offer a broader therapeutic range. Meanwhile, simultaneous targeting of CD19 and BCMA on the cell surface can reduce the possibility of antigen escape caused by the downregulation or loss of a single surface antigen.

The chimeric antigen receptor targeting CD19 and BCMA provided in the present application can have one or more effects selected from the following: (1) excellent expression efficiency and an appropriate copy number; (2) cells expressing the chimeric antigen receptor having a significant target cell killing effect; (3) cells expressing the chimeric antigen receptor having a sustained target cell killing effect; (4) cells expressing the chimeric antigen receptor having a significant cell activation capacity; and (5) cells expressing the chimeric antigen receptor having a significant cytokine secretion capacity.

In one aspect, the present application provides a chimeric antigen receptor (CAR), which comprises an extracellular antigen-binding domain that comprises a first antigen-binding domain and a second antigen-binding domain, wherein the first antigen-binding domain binds to CD19, and the second antigen-binding domain binds to BCMA; and:
1) the first antigen-binding domain comprises a first heavy chain variable region (VH) and a first light chain variable region (VL), wherein the first heavy chain variable region comprises the amino acid sequence set forth in SEQ ID NO: 3; and the first light chain variable region comprises the amino acid sequence set forth in SEQ ID NO: 1; and
2) the second antigen-binding domain comprises a second heavy chain variable region (VH), wherein the second heavy chain variable region comprises the amino acid sequence set forth in SEQ ID NO: 2.

In certain embodiments, the extracellular antigen-binding domain further comprises a linker linking the first heavy chain variable region, the first light chain variable region, and the second heavy chain variable region.

In certain embodiments, the linker comprises a β stranded linker and/or a G4S linker.

In certain embodiments, the G4S linker comprises a G4S3 linker and/or a G4S2 linker.

In certain embodiments, the β stranded linker comprises an amino acid sequence set forth in SEQ ID NO: 6 or SEQ ID NO: 7.

In certain embodiments, the G4S3 linker comprises the amino acid sequence set forth in SEQ ID NO: 4.

In certain embodiments, the G4S2 linker comprises the amino acid sequence set forth in SEQ ID NO: 5.

In certain embodiments, the extracellular antigen-binding domain comprises, from the N-terminus to the C-terminus:
1) the first heavy chain variable region, the linker, the first light chain variable region, the linker, and the second heavy chain variable region;
2) the first heavy chain variable region, the linker, the second heavy chain variable region, the linker, and the first light chain variable region;
3) the second heavy chain variable region, the linker, the first light chain variable region, the linker, and the first heavy chain variable region;
4) the second heavy chain variable region, the linker, the first heavy chain variable region, the linker, and the first light chain variable region;
5) the first light chain variable region, the linker, the second heavy chain variable region, the linker, and the first heavy chain variable region; or
6) the first light chain variable region, the linker, the first heavy chain variable region, the linker, and the second heavy chain variable region.

In certain embodiments, the extracellular antigen-binding domain comprises, from the N-terminus to the C-terminus:
1) the first light chain variable region, the G4S2 linker, the second heavy chain variable region, the G4S2 linker, and the first heavy chain variable region;
2) the first light chain variable region, the G4S3 linker, the second heavy chain variable region, the G4S3 linker, and the first heavy chain variable region;
3) the first light chain variable region, the β stranded linker, the second heavy chain variable region, the β stranded linker, and the first heavy chain variable region; or
4) the second heavy chain variable region, the G4S3 linker, the first light chain variable region, the G4S3 linker, and the first heavy chain variable region.

In certain embodiments, the extracellular antigen-binding domain comprises an amino acid sequence set forth in any one of SEQ ID NOs: 8-11.

In certain embodiments, the CAR further comprises a hinge region, a transmembrane domain, a co-stimulatory domain, and/or a signaling domain.

In certain embodiments, the CAR comprises, from the N-terminus to the C-terminus, the extracellular antigen-binding domain, the transmembrane domain, the hinge region, the co-stimulatory domain, and the signaling domain.

In certain embodiments, the hinge region comprises a hinge region derived from one or more proteins in the following group: CD28, IgG1, IgG4, IgD, CD137, CD4, CD27, CD7, CD8, PD-1, ICOS, OX40, NKG2D, NKG2C, FcεRIγ, BTLA, GITR, DAP10, TIM1, SLAM, CD30, and LIGHT.

In certain embodiments, the hinge region comprises a CD8 hinge region.

In certain embodiments, the transmembrane domain comprises a transmembrane domain derived from one or more proteins in the following group: CD8, CD28, CD3e, CD3ε, CD137, CD4, CD27, CD7, PD-1, TRAC, TRBC, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, CD154, TIM1, CD226, DR3, CD45, CD80, CD86, CD9, CD16, CD22, CD33, CD37, CD64, and SLAM.

In certain embodiments, the transmembrane domain comprises a CD8 transmembrane domain.

In certain embodiments, the co-stimulatory domain comprises a co-stimulatory domain derived from one or more proteins in the following group: CD28, 4-1BB, CD27, CD2, CD7, CD8A, CD8B, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88.

In certain embodiments, the co-stimulatory domain comprises a 4-1BB co-stimulatory domain.

In certain embodiments, the signaling domain comprises a signaling domain derived from a protein in the following group: CD3ζ, CD3δ, CD3γ, CD3ε, CD79a, CD79b, FceRIγ, FceRIβ, FcγRIIa, bovine leukemia virus gp30, Epstein-Barr virus (EBV) LMP2A, simian immunodeficiency virus PBj14 Nef, DAP10, DAP-12, and a domain protein comprising at least one ITAM.

In certain embodiments, the signaling domain comprises a CD3ζ signaling domain.

In certain embodiments, the CAR comprises an amino acid sequence set forth in any one of SEQ ID NOs: 12-15.

In another aspect, the present application provides an isolated nucleic acid molecule, wherein the isolated nucleic acid molecule encodes the CAR described in the present application.

In certain embodiments, the nucleic acid molecule comprises a nucleotide sequence set forth in any one of SEQ ID NOs: 16-19.

In another aspect, the present application provides a vector, which comprises the isolated nucleic acid molecule described in the present application.

In certain embodiments, the vector comprises a viral vector.

In certain embodiments, the vector comprises a lentiviral vector.

In another aspect, the present application provides a cell, which comprises and/or expresses the CAR described in the present application, the isolated nucleic acid molecule described in the present application, and/or the vector described in the present application.

In certain embodiments, the cell comprises an immune effector cell.

In certain embodiments, the cell comprises a T cell, a B cell, a natural killer cell (NK cell), a macrophage, an NKT cell, a γδ T cell, a monocyte, a dendritic cell, a granulocyte, a lymphocyte, a leukocyte, and/or a peripheral blood mononuclear cell.

In certain embodiments, the cell comprises a T cell.

In certain embodiments, the cell comprises a human cell.

In another aspect, the present application provides a pharmaceutical composition, which comprises the CAR described in the present application, the isolated nucleic acid molecule described in the present application, the vector described in the present application, and/or the cell described in the present application, and optionally a pharmaceutically acceptable carrier.

In another aspect, the present application provides use of the CAR described in the present application, the isolated nucleic acid molecule described in the present application, the vector described in the present application, and/or the cell described in the present application in preparing a medicament for treating a disease or disorder associated with expression of CD19 and/or BCMA.

In another aspect, the present application provides use of the CAR described in the present application, the isolated nucleic acid molecule described in the present application, the vector described in the present application, the cell described in the present application, and/or the pharmaceutical composition described in the present application in treating a disease or disorder associated with expression of CD19 and/or BCMA.

In another aspect, the present application provides a method for treating a disease or disorder associated with expression of CD19 and/or BCMA, which comprises administering to a subject in need an effective amount of the CAR described in the present application, the isolated nucleic acid molecule described in the present application, the vector described in the present application, the immune effector cell described in the present application, and/or the pharmaceutical composition described in the present application.

In certain embodiments, the disease or disorder comprises a tumor, a hematological disease, and/or an autoimmune disease.

In certain embodiments, the disease or disorder comprises a solid tumor and a non-solid tumor.

In certain embodiments, the disease comprises lymphoma and/or leukemia.

In certain embodiments, the disease comprises multiple myeloma, diffuse large B cell lymphoma, mantle cell lymphoma, small lymphocytic lymphoma, follicular lymphoma, acute myeloid leukemia, chronic myeloid leukemia, acute lymphocytic leukemia, chronic lymphocytic leukemia, non-Hodgkin's lymphoma, Hodgkin's lymphoma, megakaryocytic leukemia, Burkitt lymphoma, anaplastic large cell lymphoma, and/or mucosa-associated lymphoid tissue lymphoma.

In certain embodiments, the disease comprises chronic Guillain-Barré syndrome, systemic lupus erythematosus, lupus nephritis, rheumatoid arthritis, Sjögren's syndrome, mixed connective tissue disease, multiple sclerosis, myasthenia gravis, demyelinating diseases, autoimmune hemolysis, idiopathic thrombocytopenic purpura, and/or idiopathic leukopenia.

Other aspects and advantages of the present application will be readily apparent to those skilled in the art from the following detailed description. Only exemplary embodiments of the present application have been shown and described in the following detailed description. As those skilled in the art will recognize, the content of the present application enables those skilled in the art to make changes in the specific embodiments disclosed without departing from the spirit and scope of the invention to which the present application relates. Accordingly, the drawings and descriptions in the specification of the present application are provided only for the purpose of illustration rather than limitation.

### BRIEF DESCRIPTION OF THE DRAWINGS

The specific features of the invention to which the present application relates are set forth in the appended claims. The features and advantages of the invention to which the present application relates can be better understood with reference to the exemplary embodiments and drawings described in detail below. A brief description of the drawings is as follows:
FIGs. 1A-1B show the proliferation of T cells expressing the chimeric antigen receptor described in the present application.
FIGs. 2A-2F show the killing effect of T cells expressing the chimeric antigen receptor described in the present application on tumor cells.
FIGs. 3A-3E show the secretion of cytokines by T cells expressing the chimeric antigen receptor described in the present application co-cultured with tumor cells.

### DETAILED DESCRIPTION

The embodiments of the invention of the present application are described below with reference to specific examples, and those skilled in the art can easily understand other advantages and effects of the invention of the present application from the disclosure of this specification.

### Definitions of Terms

In the present application, the term "chimeric antigen receptor (CAR)" generally refers to an artificial cell receptor engineered to be capable of being expressed on immune effector cells and specifically binding to an antigen. CAR can be used as a therapy for adoptive cell transfer. In the present application, CAR may comprise an extracellular domain, a transmembrane domain, and an intracellular domain. CAR may further comprise a signal peptide. The extracellular domain of CAR is generally capable of binding to a target antigen. For example, the extracellular domain of the CAR may comprise an antigen-binding domain and a hinge region, and the intracellular domain of the CAR may comprise a co-stimulatory domain and a signaling domain.

In the present application, the term "bispecific" generally means that a chimeric antigen receptor can bind to at least two targets and has specificity for two different targets. For example, bispecific CAR may comprise two antigen-binding domains, each of which is specific for a different antigenic determinant. For example, the two targets may be located on the same cell surface or on different cell surfaces.

In the present application, the term "extracellular antigen-binding domain" or "antigen-binding domain" generally refers to a domain capable of binding to a target antigen. The antigen-binding domain may comprise an antibody or an antigen-binding fragment thereof capable of specifically binding. The antigen-binding domain may be of natural, synthetic, semi-synthetic, or recombinant origin. For example, the antigen-binding domain may comprise a single-chain antibody or an antigen-binding fragment thereof. For example, the antigen-binding domain may comprise an scFv. For example, the antigen-binding domain may comprise a single-domain antibody or an antigen-binding fragment thereof.

In the present application, the term "antibody" generally refers to an immunoglobulin or a fragment thereof or a derivative thereof, and encompasses any polypeptide that comprises an antigen-binding site, regardless of whether it is produced *in vitro* or *in vivo*. The term comprises, but is not limited to, polyclonal, monoclonal, monospecific, multispecific, nonspecific, humanized, single-chain, chimeric, synthetic, recombinant, hybrid, mutated, and grafted antibodies. The term "antibody" may also comprise antibody fragments such as Fab, F(ab')2, Fv, scFv, Fd, dAb, and other antibody fragments that retain antigen-binding functions. In the present application, the term "heavy chain variable region" or "VH" generally refers to a region in an antibody heavy chain structure comprising heavy chain complementarity-determining regions CDR1, CDR2, and CDR3 and framework regions FR1, FR2, FR3, and FR4. The heavy chain variable region described in the present application may comprise a heavy chain variable region of a VHH antibody. In the present application, when describing the heavy chain variable region of a VHH antibody, VHH may be used to refer thereto. In the present application, the term "light chain variable region" or "VL" generally refers to a region in an antibody light chain structure comprising light chain complementarity-determining regions CDR1, CDR2, CDR3 and framework regions FR1, FR2, FR3, and FR4.

In the present application, the term "linker" generally refers to a moiety or group of moieties that join or link 2 or more discrete, separate monomeric domains. The linker described in the present application may comprise peptide linkers of natural and/or synthetic origin, which may consist of a linear amino acid chain. In the present application, different domains of CAR may be linked by a linker, and a sub-region in a certain domain of CAR may also be linked by a linker. The linker described in the present application may comprise natural linkers and artificially designed linkers. In certain embodiments, the linker described in the present application may be a linker present in a natural protein structure or a functional variant thereof. In certain embodiments, the linker described in the present application may be a β stranded linker. In certain embodiments, the linker described in the present application may be a G4S linker comprising the amino acid sequence (Gly-Gly-Gly-Ser)n, wherein n is a positive integer equal to or greater than 1. For example, n = 1, n = 2, n = 3, n = 4, n = 5, n = 6, n = 7, n = 8, n = 9, and n = 10. In the present application, the G4S3 linker generally refers to a linker comprising the amino acid sequence (Gly-Gly-Gly-Ser)n, wherein n = 3; and the G4S2 linker generally refers to a linker comprising the amino acid sequence (Gly-Gly-Gly-Ser)n, wherein n = 2. In the present application, the term "β-stranded linker" generally refers to a structural element linking two β-sheets. A β-sheet is one type of protein secondary structure and refers to parallel or antiparallel chain-like structures formed by amino acid chains. The β-stranded linker described in the present application may comprise an amino acid sequence set forth in SEQ ID NO: 6 or SEQ ID NO: 7.

In the present application, the term "transmembrane domain" is used interchangeably with "transmembrane region (TM for short)", and generally refers to a domain of a peptide, polypeptide, or protein that is capable of spanning the cytoplasmic membrane. In CAR, the hinge region enables the extracellular antigen-binding domain to be fused to the intracellular domain, and enables CAR to be anchored to the plasma membrane of an immune effector cell. The transmembrane region in the CAR described in the present application may be obtained from a natural protein, or may be obtained synthetically, semi-synthetically, or recombinantly. The transmembrane domain in the CAR described in the present application may be derived from a transmembrane domain in a natural transmembrane protein, a functionally active fragment thereof, a truncation thereof, and/or a mutant variant thereof. In the present application, the term "CD8 transmembrane domain" generally refers to a transmembrane domain of CD8, a functionally active fragment thereof, a truncation thereof, and/or a mutant variant thereof.

In the present application, the term "hinge region" generally refers to a domain in a chimeric antigen receptor that positions the antigen-binding domain away from the surface of effector cells to enable proper cell-to-cell contact, antigen binding, and activation. In the present application, the hinge region may be located between the binding domain and the transmembrane domain. The hinge region may be from a natural source, a synthetic source, a semi-synthetic source, or a recombinant source. In the present application, the term "CD8 hinge region" generally refers to any polypeptide comprising an amino acid sequence having sequence identity or similarity to a naturally occurring CD8 hinge region sequence.

In the present application, the term "intracellular domain" is used interchangeably with "intracellular region", and generally refers to an intracellular portion within a molecule. For example, the intracellular domain of the CAR described in the present application may comprise a co-stimulatory domain and a signaling domain. In the present application, the term "co-stimulatory domain" generally refers to a portion of CAR capable of providing a second signal required for activation of an immune effector cell. The co-stimulatory domain in CAR may be from a natural co-stimulatory molecule on the surface of an immune effector cell. In the present application, the term "signaling domain" generally refers to a portion of CAR that participates in transducing effective CAR-binding antigen polypeptide information into the interior of an immune effector cell to trigger effector cell functions, such as activation, cytokine production, proliferation, and cytotoxic activity, including release of a cytotoxic factor to a CAR-bound target cell or other cellular responses elicited by an antigen bound to an extracellular CAR domain.

In the present application, the term "vector" generally refers to a nucleic acid molecule capable of transporting another nucleic acid to which it is linked. One type of vector is a "plasmid," which refers to a circular, double stranded DNA loop into which an additional DNA segment can be ligated. Another type of vector is a viral vector, wherein an additional DNA segment can be ligated into the viral genome. Certain vectors are capable of autonomous replication in a host cell into which they are introduced (e.g., bacterial vectors having a bacterial origin of replication and episomal mammalian vectors). In the present application, the term "viral vector" generally refers to a non-wild-type recombinant viral particle that serves as a gene delivery vector and comprises a recombinant viral genome packaged within a viral capsid. The types of viruses used as vectors may comprise, but are not limited to, retroviruses (including lentiviruses), adenoviruses, adeno-associated viruses (AAVs), herpes viruses (such as herpes simplex virus), poxviruses, baculoviruses, papillomaviruses, and papovaviruses (such as SV40).

In the present application, the term "immune effector cell" generally refers to a cell involved in an immune response, e.g., promoting an immune effector response. Examples of immune effector cells comprise, but are not limited to, T cells, B cells, natural killer (NK) cells, NKT cells, mast cells, granulocytes, monocytes, lymphocytes, and macrophages. The term also comprises engineered immune cells, such as immune cells genetically modified by the addition of the exogenous genetic material in the form of DNA or RNA to the total genetic material of the cell.

In the present application, the terms "T lymphocyte" and "T cell" are used interchangeably and generally refer to lymphocytes including thymocytes, immature T lymphocytes, mature T lymphocytes, resting T lymphocytes, or activated T lymphocytes. The T cell may be a helper T (Th) cell, such as a helper T1 (Th1) cell or a helper T2 (Th2) cell. The T cell may be a helper T cell (HTL; CD4+ T cell), a CD4+ T cell, a cytotoxic T cell (CTL; CD8+ T cell), a CD4+CD8+ T cell, or a CD4-CD8- T cell. Alternatively, the T lymphocyte may comprise a naive T cell and a memory T cell.

In the present application, the term "killing capacity" generally refers to the capacity to kill cells achieved by contacting the cells with an effective amount of an antibody, an immunoconjugate, a bispecific/multispecific molecule, or a composition. The method may comprise killing cells positive for antigen expression, optionally in the presence of effector cells, e.g., by CDC, apoptosis, ADCC, phagocytosis, or a combination of two or more of these mechanisms.

In the present application, the term "cytokine" generally refers to a protein released by a cell population that acts as an intercellular regulator for another cell. For example, the cytokines may comprise interleukins (ILs), interferons (IFNs), chemokines, tumor necrosis factors (TNFs), and transforming growth factors (TGFs).

In the present application, the term "pharmaceutical composition" generally refers to a formulation in a form that allows the biological activity of the active ingredient to be effective, and it does not contain additional ingredients that are unacceptably toxic to a subject to which the formulation is to be administered. In the present application, the term "pharmaceutically acceptable carrier" generally comprises pharmaceutically acceptable vehicles, excipients, or stabilizers.

In the present application, the term "subject" generally refers to an animal, typically a mammal, such as a human, a non-human primate (an ape, a gibbon, a gorilla, a chimpanzee, an orangutan, and a macaque), a domestic animal (a dog and a cat), a farm animal (poultry such as a chicken and a duck, a horse, a cow, a goat, a sheep, a pig), and a laboratory animal (a mouse, a rat, a rabbit, and a guinea pig). Human subjects may comprise fetal, neonatal, infant, juvenile, and adult subjects. The subject may comprise an animal disease model.

In the present application, the term "effective amount" or "effective dose" generally refers to an amount sufficient to achieve, or at least partially achieve, a desired effect. A "therapeutically effective amount" or "therapeutically effective dose" of a drug or therapeutic agent is generally any amount of the drug that promotes the regression of a disease (as evidenced by a decrease in the severity of the symptoms of the disease, an increase in the frequency and duration of the asymptomatic phase of the disease, or the prevention of the damage or disability due to the development of the disease) when used alone or in combination with another therapeutic agent.

In the present application, the term "tumor" generally refers to a physiological condition characterized by unregulated cell growth. In the present application, the tumor may be benign or malignant. In the present application, the tumor may be a solid tumor or a non-solid tumor. In the present application, the terms "cancer", "cancerous", "cell proliferative disorder", "proliferative disorder", and "tumor" are not mutually exclusive when referred to herein. In certain cases, the term "cancer" is used to refer to a malignant tumor.

In the present application, the term "hematological disease" generally refers to a pathological state that primarily or mainly involves the blood and hematopoietic organs. The hematological disease may involve abnormal production, abnormal function, or destruction of blood cells (including erythrocytes, leukocytes, and platelets), as well as an impairment of coagulation and bleeding mechanisms.

In the present application, the term "autoimmune disease" is generally an impairment or disorder resulting from an autoimmune response mediated by an antibody against a self-antigen. Autoimmune diseases cause the inappropriate production and/or overproduction of autoantibodies against self-antigens. Autoimmune diseases are systemic diseases that may occur in almost all sites of the body, including the nervous system, gastrointestinal system, endocrine system, skin, skeletal system, and vascular tissue.

In the present application, the term "treat", "treating", or "treatment" comprises not only the treatment of a disease, but also generally the slowing or reversing of disease progression, the prevention or slowing of the onset of one or more symptoms associated with the disease, the reduction and/or alleviation of one or more symptoms associated with the disease, the reduction of the severity and/or duration of the disease and/or any symptoms associated therewith, and/or the prevention of further increases in the severity of the disease and/or any symptoms associated therewith, as well as the prevention, reduction, or reversal of any physiological damage arising from the disease and any pharmacological effects that are generally beneficial to a treated patient.

In the present application, the term "comprise" or "comprising" generally refers to the inclusion of the explicitly specified features without excluding other elements.

In the present application, the term "about" generally means varying by 0.5%-10% above or below the stated value, for example, varying by 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below the stated value.

### Detailed Description of the Present Disclosure

In one aspect, the present application provides a chimeric antigen receptor (CAR), which has a capacity to simultaneously target CD19 and BCMA. In certain embodiments, the CAR has bispecificity for CD19 and BCMA.

In the present application, the chimeric antigen receptor comprises an extracellular antigen-binding domain that comprises a first antigen-binding domain and a second antigen-binding domain, wherein the first antigen-binding domain binds to CD19, and the second antigen-binding domain binds to BCMA.

In the present application, the first antigen-binding domain may bind to or associate with CD19 with Ka (i.e., an equilibrium association constant for binding interaction, expressed in 1/M) of greater than or equal to about 10⁵M⁻¹ (e.g., greater than or equal to about 10⁵M⁻¹, greater than or equal to about 10⁶M⁻¹, greater than or equal to about 10⁷M⁻¹, greater than or equal to about 10⁸M⁻¹, greater than or equal to about 10⁹M⁻¹, greater than or equal to about 10¹⁰M⁻¹, greater than or equal to about 10¹¹M⁻¹, greater than or equal to about 10¹²M⁻¹, greater than or equal to about 10¹³M⁻¹, or greater), or bind to or associate with CD19 with an equilibrium dissociation constant Kd of less than or equal to about 10⁻⁵M (e.g., less than or equal to about 10⁻⁵M, less than or equal to about 10⁻⁶M, less than or equal to about 10⁻⁷M, less than or equal to about 10⁻⁸M, less than or equal to about 10⁻⁹M, less than or equal to about 10⁻¹⁰M, less than or equal to about 10⁻¹¹M, less than or equal to about 10⁻¹²M, less than or equal to about 10⁻¹³M, or less).

In the present application, the second antigen-binding domain may bind to or associate with BCMA with Ka (i.e., an equilibrium association constant for binding interaction, expressed in 1/M) of greater than or equal to about 10⁵M⁻¹ (e.g., greater than or equal to about 10⁵M⁻¹, greater than or equal to about 10⁶M⁻¹, greater than or equal to about 10⁷M⁻¹, greater than or equal to about 10⁸M⁻¹, greater than or equal to about 10⁹M⁻¹, greater than or equal to about 10¹⁰M⁻¹, greater than or equal to about 10¹¹M⁻¹, greater than or equal to about 10¹²M⁻¹, greater than or equal to about 10¹³M⁻¹, or greater), or bind to or associate with BCMA with an equilibrium dissociation constant Kd of less than or equal to about 10⁻⁵M (e.g., less than or equal to about 10⁻⁵M, less than or equal to about 10⁻⁶M, less than or equal to about 10⁻⁷M, less than or equal to about 10⁻⁸M, less than or equal to about 10⁻⁹M, less than or equal to about 10⁻¹⁰M, less than or equal to about 10⁻¹¹M, less than or equal to about 10⁻¹²M, less than or equal to about 10⁻¹³M, or less).

In the present application, the affinity of the first antigen-binding domain for CD19 and the second antigen-binding domain for BCMA may be determined by conventional techniques in the art, for example, by competitive ELISA (enzyme-linked immunosorbent assay), or by binding association, or by displacement assay using a labeled ligand, or by using surface plasmon resonance apparatus (e.g., BiacoreT100, available from Biacore, Inc., Piscataway, NJ) or optical biosensor techniques.

In certain embodiments, the first antigen-binding domain comprises a first heavy chain variable region (VH) and a first light chain variable region (VL).

In certain embodiments, the first heavy chain variable region comprises the amino acid sequence set forth in SEQ ID NO: 3 or a functional variant thereof, wherein the functional variant is selected from the group consisting of: a protein or polypeptide with one or more amino acids substituted, deleted, or added in the amino acid sequence set forth in SEQ ID NO: 3; and a protein or polypeptide having 90% or higher (e.g., at least about 90%, about 91%, about 92%, about 93%, about 94%, about 95%, about 96%, about 97%, about 98%, about 99%, or higher) sequence homology to the amino acid sequence set forth in SEQ ID NO: 3.

In certain embodiments, the first light chain variable region comprises the amino acid sequence set forth in SEQ ID NO: 1 or a functional variant thereof, wherein the functional variant is selected from the group consisting of: a protein or polypeptide with one or more amino acids substituted, deleted, or added in the amino acid sequence set forth in SEQ ID NO: 1; and a protein or polypeptide having 90% or higher (e.g., at least about 90%, about 91%, about 92%, about 93%, about 94%, about 95%, about 96%, about 97%, about 98%, about 99%, or higher) sequence homology to the amino acid sequence set forth in SEQ ID NO: 1.

In certain embodiments, the first heavy chain variable region comprises the amino acid sequence set forth in SEQ ID NO: 3, and the first light chain variable region comprises the amino acid sequence set forth in SEQ ID NO: 1.

In certain embodiments, the second antigen-binding domain comprises a second heavy chain variable region (VH). In certain embodiments, the second antigen-binding domain comprises the amino acid sequence set forth in SEQ ID NO: 2 or a functional variant thereof, wherein the functional variant is selected from the group consisting of: a protein or polypeptide with one or more amino acids substituted, deleted, or added in the amino acid sequence set forth in SEQ ID NO: 2; and a protein or polypeptide having 90% or higher (e.g., at least about 90%, about 91%, about 92%, about 93%, about 94%, about 95%, about 96%, about 97%, about 98%, about 99%, or higher) sequence homology to the amino acid sequence set forth in SEQ ID NO: 2.

In certain embodiments, the extracellular antigen-binding domain of the chimeric antigen receptor comprises a first antigen-binding domain and a second antigen-binding domain, without being limited to any linking mode or linking order. In certain embodiments, the first antigen-binding domain is directly or indirectly linked to the second antigen-binding domain. For example, the first antigen-binding domain is linked to the second antigen-binding domain via a linker.

In certain embodiments, the extracellular antigen-binding domain of the chimeric antigen receptor comprises the first antigen-binding domain and the second antigen-binding domain, wherein the first antigen-binding domain comprises a first heavy chain variable region and a first light chain variable region, and the second antigen-binding domain comprises a second heavy chain variable region. In certain embodiments, the extracellular antigen-binding domain comprises the first heavy chain variable region, the first light chain variable region, and the second heavy chain variable region, without being limited to linking mode or linking order. For example, the first heavy chain variable region, the first light chain variable region, and the second heavy chain variable region are linked via a linker. For example, in the extracellular antigen-binding domain, the order of the variable regions from the N-terminus to the C-terminus may be sequentially: 1) the first heavy chain variable region, the first light chain variable region, and the second heavy chain variable region; 2) the first heavy chain variable region, the second heavy chain variable region, and the first light chain variable region; 3) the first light chain variable region, the first heavy chain variable region, and the second heavy chain variable region; 4) the first light chain variable region, the second heavy chain variable region, and the first heavy chain variable region; 5) the second heavy chain variable region, the first heavy chain variable region, and the first light chain variable region; or 6) the second heavy chain variable region, the first light chain variable region, and the first heavy chain variable region.

In certain embodiments, the extracellular antigen-binding domain of the chimeric antigen receptor comprises a linker linking the first heavy chain variable region, the first light chain variable region, and the second heavy chain variable region.

In certain embodiments, the linker comprises a β stranded linker and/or a G4S linker. For example, the β stranded linker may comprise, but is not limited to, an amino acid sequence set forth in SEQ ID NO: 6 or SEQ ID NO: 7. For example, the G4S linker may comprise the amino acid sequence (Gly-Gly-Gly-Ser)n, wherein n is a positive integer equal to or greater than 1. For example, n = 1, n = 2, n = 3, n = 4, n = 5, n = 6, n = 7, n = 8, n = 9, and n = 10. In certain embodiments, the G4S linker may comprise a G4S2 linker comprising the amino acid sequence (Gly-Gly-Gly-Ser)n, wherein n = 2. In certain embodiments, the G4S linker may comprise a G4S3 linker comprising the amino acid sequence (Gly-Gly-Gly-Ser)n, wherein n = 3. In certain embodiments, the G4S linker may comprise, but is not limited to, an amino acid sequence set forth in SEQ ID NO: 4 or SEQ ID NO: 5.

In certain embodiments, the extracellular antigen-binding domain of the chimeric antigen receptor comprises one, two, or more linkers. When the extracellular antigen-binding domain comprises two or more linkers, they may be identical, partially identical, or completely different linkers.

In certain embodiments, the extracellular antigen-binding domain of the chimeric antigen receptor comprises, from the N-terminus to the C-terminus:
1) the first heavy chain variable region, the linker, the first light chain variable region, the linker, and the second heavy chain variable region;
2) the first heavy chain variable region, the linker, the second heavy chain variable region, the linker, and the first light chain variable region;
3) the second heavy chain variable region, the linker, the first light chain variable region, the linker, and the first heavy chain variable region;
4) the second heavy chain variable region, the linker, the first heavy chain variable region, the linker, and the first light chain variable region;
5) the first light chain variable region, the linker, the second heavy chain variable region, the linker, and the first heavy chain variable region; or
6) the first light chain variable region, the linker, the first heavy chain variable region, the linker, and the second heavy chain variable region,
wherein the linker may be independently selected from a β stranded linker, a G4S2 linker, and a G4S3 linker.

In certain embodiments, the extracellular antigen-binding domain of the chimeric antigen receptor comprises, from the N-terminus to the C-terminus:
1) the first light chain variable region, the G4S2 linker, the second heavy chain variable region, the G4S2 linker, and the first heavy chain variable region;
2) the first light chain variable region, the G4S3 linker, the second heavy chain variable region, the G4S3 linker, and the first heavy chain variable region;
3) the first light chain variable region, the β stranded linker, the second heavy chain variable region, the β stranded linker, and the first heavy chain variable region; or
4) the second heavy chain variable region, the G4S3 linker, the first light chain variable region, the G4S3 linker, and the first heavy chain variable region.

In certain embodiments, the extracellular antigen-binding domain comprises an amino acid sequence set forth in any one of SEQ ID NOs: 8-11 or a functional variant thereof, wherein the functional variant is selected from the group consisting of: a protein or polypeptide with one or more amino acids substituted, deleted, or added in an amino acid sequence set forth in any one of SEQ ID NOs: 8-11; and a protein or polypeptide having 90% or higher (e.g., at least about 90%, about 91%, about 92%, about 93%, about 94%, about 95%, about 96%, about 97%, about 98%, about 99%, or higher) sequence homology to an amino acid sequence set forth in any one of SEQ ID NOs: 8-11.

In the present application, the chimeric antigen receptor described in the present application may further comprise optionally a signal peptide, a hinge region, a transmembrane domain, a co-stimulatory domain, and/or a signaling domain. In certain embodiments, a linker may be comprised between the domains of the chimeric antigen receptor.

In the present application, the extracellular antigen-binding domain of the chimeric antigen receptor may be linked to the transmembrane domain via a hinge region. For example, the chimeric antigen receptor may comprise one or more of the hinge regions between the binding domain and the transmembrane domain. For example, the hinge region may comprise a hinge region derived from one or more proteins in the following group: CD28, IgG1, IgG4, IgD, CD137, CD4, CD27, CD7, CD8, PD-1, ICOS, OX40, NKG2D, NKG2C, FcεRIγ, BTLA, GITR, DAP10, TIM1, SLAM, CD30, and LIGHT. In certain embodiments, the chimeric antigen receptor comprises a CD8 hinge region, wherein the CD8 hinge region may comprise the amino acid sequence set forth in SEQ ID NO: 21. In the present application, the chimeric antigen receptor may comprise one or more transmembrane domains, wherein the transmembrane domain may be located at the C-terminus of the hinge region. For example, the transmembrane domain may comprise a transmembrane domain derived from one or more proteins in the following group: CD8, CD28, CD3e, CD3ε, CD137, CD4, CD27, CD7, PD-1, TRAC, TRBC, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, CD154, TIM1, CD226, DR3, CD45, CD80, CD86, CD9, CD16, CD22, CD33, CD37, CD64, and SLAM. In certain embodiments, the chimeric antigen receptor comprises a CD8 transmembrane domain, wherein the CD8 transmembrane domain may comprise the amino acid sequence set forth in SEQ ID NO: 22.

In the present application, the chimeric antigen receptor may comprise one or more co-stimulatory domains, wherein the co-stimulatory domain may be located at the C-terminus of the transmembrane domain. For example, the co-stimulatory domain may comprise a co-stimulatory domain derived from one or more proteins in the following group: CD28, 4-1BB, CD27, CD2, CD7, CD8A, CD8B, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88. In certain embodiments, the chimeric antigen receptor comprises a 4-1BB co-stimulatory domain, wherein the 4-1BB co-stimulatory domain may comprise the amino acid sequence set forth in SEQ ID NO: 23.

In the present application, the chimeric antigen receptor may comprise one or more signaling domains, wherein the signaling domain may be located at the C-terminus of the co-stimulatory domain. In the present application, the signaling domain may comprise a signaling domain derived from one or more proteins in the following group: CD3ζ, CD3δ, CD3γ, CD3ε, CD79a, CD79b, FceRIγ, FceRIβ, FcγRIIa, bovine leukemia virus gp30, Epstein-Barr virus (EBV) LMP2A, simian immunodeficiency virus PBj14 Nef, DAP10, DAP-12, and a domain protein comprising at least one ITAM. In certain embodiments, the chimeric antigen receptor comprises a CD3ζ signaling domain, wherein the CD3ζ signaling domain may comprise the amino acid sequence set forth in SEQ ID NO: 24.

In the present application, the CAR may further comprise a signal peptide. In the present application, the signal peptide may comprise a signal peptide selected from CD8 and CD45.

In certain embodiments, the chimeric antigen receptor described in the present application sequentially comprises, from the N-terminus to the C-terminus, a signal peptide, an antigen-binding domain, a hinge region, a transmembrane domain, a co-stimulatory domain, and a signaling domain. In certain embodiments, the chimeric antigen receptor described in the present application may comprise an amino acid sequence set forth in any one of SEQ ID NOs: 12-15 or a functional variant thereof, wherein the functional variant is selected from the group consisting of: a protein or polypeptide with one or more amino acids substituted, deleted, or added in an amino acid sequence set forth in any one of SEQ ID NOs: 12-15; and a protein or polypeptide having 90% or higher (e.g., at least about 90%, about 91%, about 92%, about 93%, about 94%, about 95%, about 96%, about 97%, about 98%, about 99%, or higher) sequence homology to an amino acid sequence set forth in any one of SEQ ID NOs: 12-15.

In another aspect, the present application provides an isolated nucleic acid molecule, which can encode the chimeric antigen receptor described in the present application.

In certain embodiments, the nucleic acid molecule described in the present application comprises a nucleotide sequence set forth in any one of SEQ ID NOs: 16-19 or a nucleotide sequence having 90% or higher (e.g., at least about 90%, about 91%, about 92%, about 93%, about 94%, about 95%, about 96%, about 97%, about 98%, about 99%, or higher) sequence homology to a nucleotide sequence set forth in any one of SEQ ID NOs: 16-19.

In another aspect, the present application provides a vector, which comprises a nucleic acid molecule capable of encoding the chimeric antigen receptor described in the present application. In certain embodiments, the vector described in the present application is capable of expressing the chimeric antigen receptor described in the present application in a cell.

In the present application, the vector may further comprise one or more of an origin of replication, a selection cassette, a promoter, an enhancer, a translation initiation signal (a Shine Dalgarno sequence or a Kozak sequence), an intron, a polyadenylation sequence, and 5' and 3' untranslated regions. In the present application, the vector may be selected from a plasmid, a phage, an artificial chromosome (e.g., a yeast artificial chromosome YAC), and a viral vector. The viral vector may comprise, but is not limited to, a lentiviral vector, an adenoviral vector, and a retroviral vector.

In another aspect, the present application provides a cell, which comprises the chimeric antigen receptor described in the present application, the nucleic acid molecule described in the present application, and/or the vector described in the present application. For example, the cell provided in the present application may be an artificially modified cell.

In the present application, the cell may comprise an immune effector cell. For example, the immune effector cell may comprise a T cell, a B cell, a natural killer cell (NK cell), a macrophage, an NKT cell, a γδ T cell, a monocyte, a dendritic cell, a granulocyte, a lymphocyte, a leukocyte, and/or a peripheral blood mononuclear cell. For example, the immune effector cell is a T cell.

In the present application, the method may comprise a step of obtaining the immune effector cell from a subject. For example, the T lymphocytes may be obtained from peripheral blood mononuclear cells, bone marrow, lymph node tissues, umbilical cord blood, thymus tissues, tissues from an infection site, ascites, pleural effusion, spleen tissues, and tumors. In addition, the method may further comprise a step of selecting a specific cell subset from the immune effector cells. For example, specific T cell subsets may be selected based on specific expression of CD3, CD28, CD4, CD8, CD45RA, and CD45RO. In certain embodiments, the cell comprises a human cell. For example, the cell described in the present application may be a modified human cell.

In another aspect, the present application provides a method for preparing the cell described in the present application, which comprises introducing the chimeric antigen receptor described in the present application, the nucleic acid molecule described in the present application, and/or the vector described in the present application into the cell.

In another aspect, the present application provides a composition, which comprises the chimeric antigen receptor described in the present application, the nucleic acid molecule described in the present application, the vector described in the present application, and/or the cell described in the present application. In certain embodiments, the composition is a pharmaceutical composition, which comprises the chimeric antigen receptor described in the present application, the nucleic acid molecule described in the present application, the vector described in the present application, and/or the cell described in the present application, and optionally a pharmaceutically acceptable carrier.

In the present application, the pharmaceutically acceptable carrier may be selected from the group consisting of: an excipient, a glidant, a sweetener, a diluent, a preservative, a dye/colorant, a flavor enhancer, a surfactant, a wetting agent, a dispersant, a suspending agent, a stabilizer, an isotonic agent, a solvent, a surfactant, and an emulsifier.

In certain embodiments, a biologically active ingredient in the pharmaceutical composition described in the present application is in an effective amount. The effective amount may be the lowest dose required to achieve a beneficial or desired prophylactic or therapeutic effect. For example, the effective amount may be influenced by factors such as the degree of the disease, age, body weight, and sex of the subject.

In another aspect, the present application provides use of the chimeric antigen receptor described in the present application, the nucleic acid molecule described in the present application, the vector described in the present application, and/or the cell described in the present application in preparing a medicament.

In certain embodiments, the medicament is used for treating a disease or disorder associated with expression of CD19 and/or BCMA.

In another aspect, the present application provides use of the chimeric antigen receptor described in the present application, the nucleic acid molecule described in the present application, the vector described in the present application, the cell described in the present application, and/or the pharmaceutical composition described in the present application in treating a disease or disorder associated with expression of CD19 and/or BCMA.

In another aspect, the present application provides a method for treating a disease or disorder associated with expression of CD19 and/or BCMA, which comprises administering to a subject in need an effective amount of the chimeric antigen receptor described in the present application, the nucleic acid molecule described in the present application, the vector described in the present application, the cell described in the present application, and/or the pharmaceutical composition described in the present application.

In the present application, the disease or disorder associated with expression of CD19 and/or BCMA comprises a disorder involving or mediated by CD19-positive and/or BCMA-positive cells.

In certain embodiments, the disease or disorder associated with expression of CD19 and/or BCMA comprises a tumor associated with expression of CD19 and/or BCMA. For example, the tumor comprises a solid tumor and a non-solid tumor. In certain embodiments, the disease or disorder associated with expression of CD19 and/or BCMA comprises a hematological disease associated with expression of CD19 and/or BCMA. In certain embodiments, the disease or disorder associated with expression of CD19 and/or BCMA comprises an autoimmune disease associated with expression of CD19 and/or BCMA.

For example, the disease or disorder associated with expression of CD19 and/or BCMA may comprise lymphoma and/or leukemia associated with expression of CD19 and/or BCMA. For example, the disease or disorder associated with expression of CD19 and/or BCMA may comprise multiple myeloma, diffuse large B cell lymphoma, mantle cell lymphoma, small lymphocytic lymphoma, follicular lymphoma, acute myeloid leukemia, chronic myeloid leukemia, acute lymphocytic leukemia, chronic lymphocytic leukemia, non-Hodgkin's lymphoma, Hodgkin's lymphoma, megakaryocytic leukemia, Burkitt lymphoma, anaplastic large cell lymphoma, and/or mucosa-associated lymphoid tissue lymphoma.

For example, the disease or disorder associated with expression of CD19 and/or BCMA may comprise chronic Guillain-Barré syndrome, systemic lupus erythematosus, lupus nephritis, rheumatoid arthritis, Sjögren's syndrome, mixed connective tissue disease, multiple sclerosis, myasthenia gravis, demyelinating diseases, autoimmune hemolysis, idiopathic thrombocytopenic purpura, and/or idiopathic leukopenia.

Without being bound by any theory, the following examples are intended only to illustrate the chimeric antigen receptor, preparation method, use, etc., of the present application, and are not intended to limit the scope of the invention of the present application.

### EXAMPLES

The present application is further illustrated by the following specific examples. It should be understood that these examples are merely intended to illustrate the present application rather than limit the scope of the present application. Experimental methods without specified conditions in the following examples were performed according to conventional conditions in the art, such as conditions described in Sambrook and Russeii et al., Molecular Cloning: A Laboratory Manual (Third Edition) (2001), CSHL Press, or according to conditions recommended by the manufacturer. Unless otherwise stated, the experimental materials and reagents used in the following examples are commercially available.

The key materials used in the examples of the present application are shown in Tables 1-3.

**Table 1. List of key materials (plasmids)**

| Name | Manufacturer | Catalog number |
|---|---|---|
| Tryptone | OXOID | LP0042B |
| Yeast extract | OXOID | LP0021 |
| Sodium chloride | Hebei Huachen Pharmaceutical | Registration No. Y20190008765 |
| Kanamycin sulfate | Chengdu Brilliant Pharmaceuticals | Registration No. Y20190001765 |
| Sterile water for injection | Sichuan Kelun Pharmaceutical | National medical approval No. H20044405 |
| Endofree maxi plasmid kit-purification of plasmid | TIANGEN | DP117 |
| Absolute ethanol (analytical reagent) | Sinopharm | 10009218 |
| Isopropanol (analytical reagent) | Sinopharm | 80109218 |
| Glycerol | Sangon Biotech (Shanghai) | |
| Isopropanol (analytical reagent) | Sinopharm | 80109218 |

**Table 2. List of key materials (viruses)**

| Name | Manufacturer | Catalog number |
|---|---|---|
| SMM 293TII serum-free medium | Beijing Sino Biological | M293TII |
| OPM-293 CD05 Medium | Shanghai OPM Biosciences | 81075-001 |
| OPM-CHO PFF06 | Shanghai OPM Biosciences | 1265F05-001 |
| 200mM Glutamine | Self-made | N/A |
| PEI pro-GMP | Polyplus | 102000121 |
| Sterile water for injection | Sichuan Kelun Pharmaceutical | National medical approval No. H20044405 |

| 0.9% sodium chloride injection | Sichuan Kelun Pharmaceutical | National medical approval No. H20034174 |
|---|---|---|
| 20% human serum albumin (HSA) | CSL Behring, Switzerland | S20170005 |
| Trypan blue solution | Gibco | 15250-061 |
| RPMI-1640 Medium | Hyclone | SH30809.01 |
| FBS | Hyclone | SV30208.02 |
| PBS | Tianjin TBDscience | PB2004Y |

**Table 3. List of key materials (cells)**

| Name | Manufacturer | Catalog number |
|---|---|---|
| CliniMACS CD3 Reagent | Miltenyi Biotec B.V. & Co.KG | 200-070-113 |
| CliniMACS PBS/EDTA Buffer | Miltenyi Biotec B.V. & Co.KG | 200-070-025 |
| X-VivoTM15 | Lonza | BEBP02-054Q |
| TransAct | Miltenyi Biotec B.V. & Co.KG | 130-111-160 |
| LS Columns | Miltenyi Biotec B.V. & Co.KG | 130-042-401 |
| Recombinant human interleukin-2 injection | Beijing Sihuanshengwu | National medical approval No. S20040018 |
| 0.9% sodium chloride injection | Sichuan Kelun Pharmaceutical | National medical approval No. H20034174 |
| 20% human serum albumin (HSA) | CSL Behring, Switzerland | S20170005 |
| Ficoll-Paque density gradient media | Cytiva | 17144002 |
| RPMI-1640 Medium | Cytiva | SH30809.01 |
| FBS | Cytiva | SV30208.02 |
| PBS | Tianjin TBDscience | PB2004Y |
| CTS^{™} immune cell serum replacement | Gibco | A25961-02 |
| Trypan blue solution | Gibco | 15250-061 |
| 24-well plate | Corning | 3524 |
| T-25 culture flask | Corning | 430639 |
| T-75 culture flask | Corning | 430641 |
| FITC anti-human CD3 Antibody | BioLegend | 317306 |
| FITC Mouse IgG2a, κ Isotype Ctrl | BioLegend | 981902 |
| FITC-Labeled Human CD19(20-291)Protein, His Tag | ACRO | CD9-HF2H3 |
| PE-Labeled Human BCMA His Tag | ACRO | BCA-HP2H2 |
| CytoTox 96 Non-Radioactive Cytotoxin city Assay | Promega | G1780 |
| Tissue/cell genomic DNA rapid extraction kit | Biomed | DL107-01 |
| Beadstar-mPlex^{®} mouse multi-cytokine assay kit | Beadstar Biotechnology (Hainan) Co., Ltd. | BS-MC01 |

### Example 1. Construction of CD19-BCMA Dual-Target CAR Vectors

Construction of Pre Lenti EF1 CAR V2 WPmut lentiviral vector:
(1) The 5' LTR of the shuttle plasmid pRRLSIN.cPPT.PGK-GFP.WPRE (for its plasmid map, see the website www.addgene.org/12252/) is an RSV promoter. Replacing the promoter of the 5' LTR from RSV with CMV helps to efficiently transcribe the viral genome sequence. The U3 region (3' LTR Δ U3) has been removed from the original 3' LTR plasmid, a self-inactivating (SIN) design has been performed, and the 3' LTR sequence remains unchanged.
(2) The hPGK promoter driving the expression of the exogenous transgene was replaced with an EF1 promoter, and the EGFP gene was replaced with a polyclonal sequence.
(3) The ampicillin resistance gene was replaced with a kanamycin resistance gene from the pUC57-Kan (GenBank: LT671993.1) plasmid.
(4) The WPRE element of pRRLSIN.cPPT.PGK-GFP.WPRE was modified so that the truncated X protein was not expressed. Specifically, in the sequence of the wild-type WPRE element, the promoter driving the transcription of the X protein was deleted, A of the start codon ATG was changed to T, and the new sequence was named WPREmut.

The engineered shuttle plasmid described above was named Pre-Lenti-EF1a-CAR V2 WPmut (the sequence of which is set forth in SEQ ID NO: 20). On this basis, a plasmid of CAR capable of simultaneously targeting CD19 and BCMA was constructed. The CAR comprised an scFv sequence targeting CD19 and a VHH sequence targeting BCMA, which were linked via a βS linker, a (G4S)₂ linker, or a (G4S)₃ linker, respectively, as the antigen-binding domain, and the intracellular domains of 4-1BB and CD3ζ molecules as the cytoplasmic region. Different CAR sequences were constructed into the Pre-Lenti-EF1a-CAR V2 WPmut lentiviral vector to obtain CD19-BCMA dual-target CAR plasmids. The different CARs and their amino acid sequences, antigen-binding domain structures and their amino acid sequences, and the corresponding plasmids are shown in Table 4.

**Table 4. CD19-BCMA dual-target CAR plasmids**

| CAR | Antigen-binding domain structure | Plasmid Name |
|---|---|---|
| βS CAR (SEQ ID NO: 14) | CD19 VL-βS linker-BCMA VHH-βS linker-CD19 VH (SEQ ID NO: 10) | Pre-Lenti-EF1a-CAR V2 WPmut-CD19-BCMA dual-beta-CAR01 |
| (G4S)₂ CAR (SEQ ID NO: 12) | CD19 VL-(G4S)₂ linker-BCMA VHH-(G4S)₂ linker-CD19 VH (SEQ ID NO: 8) | Pre-Lenti-EF1a-CAR V2 WPmut-CD19-BCMA dual-G4S2-CAR02 |
| (G4S)₃₋₁ CAR (SEQ ID NO: 13) | CD19 VL-(G4S)₃ linker-BCMA VHH-(G4S)₃ linker-CD19 VH (SEQ ID NO: 9) | Pre-Lenti-EF1a-CAR V2 WPmut-CD19-BCMA dual-G4S3-CAR03 |
| (G4S)₃₋₂ CAR (SEQ ID NO: 15) | BCMA VHH-(G4S)₃ linker-CD19 VL-(G4S)₃ linker-CD19 VH (SEQ ID NO: 11) | Pre Lenti EF1 CAR V2 WPmut-CD19-BCMA dual-G4S3-CAR |

### Example 2. Preparation of CD19-BCMA Dual-Target CAR Lentiviruses

In this example, lentiviruses comprising a plasmid of CAR capable of simultaneously targeting CD19 and BCMA were prepared.

The plasmids comprising different CARs in Example 1 were mixed thoroughly with the packaging plasmid ZL004, the envelope plasmid ZL003, and the packaging plasmid ZL006, respectively, at a ratio of 7:5:3:5 (for the plasmids ZL004, ZL003, and ZL006, refer to Chinese Patent Application Publication No. CN111979230A), and the mixture of the above four plasmids was added to a centrifuge tube containing a 293TS basic medium. PEIpro was added to another centrifuge tube at a plasmid-to-PEIpro ratio of 1:2. After incubation of the two tubes at room temperature for 5 minutes, the mixed liquid containing PEIpro was slowly added to the mixed liquid containing the plasmids. The tube was gently shaken for thorough mixing and incubated at room temperature for 15 minutes. The transfection mixture was then added dropwise to a 293TS cell suspension with a density of 5.0-8.0 × 10⁶ cells/mL. 6-8 hours after transfection, feeding was performed using an OPM-CHO PFF06 feeding medium containing glutamine. After 48 hours, a medium supernatant was collected to obtain a crude lentivirus liquid. The crude lentivirus liquid was subjected to steps such as centrifugal filtration, enzymatic digestion, ultrafiltration concentration, and sterilization and filtration. The virus liquid was mixed thoroughly with 20% HSA at a ratio of 9:1 to obtain a lentivirus. The prepared lentivirus was aliquoted and stored at -80 °C. The transduction titers of the lentiviruses were determined by flow cytometry. The determination results of the transduction titers are shown in the table below:

**Table 5. Lentivirus transduction titers**

| Virus batch No. | Transduction titer (TU/mL) |
|---|---|
| LV2311-240507-(G4S)₂ CAR | 1.18E+08 |
| LV2311-240507-(G4S)₃₋₁ CAR | 1.02E+08 |
| LV2311-240507-(G4S)₃₋₂ CAR | 6.2E+07 |
| LV2311-240507-βs CAR | 7.75E+07 |

### Example 3. Preparation of CD19-BCMA Dual-Target CAR-T Cells

In this example, T cells expressing the CAR capable of simultaneously targeting CD19 and BCMA described in the present application were prepared.

Peripheral blood of healthy volunteers was collected and purified by the Miltenyi CD3 Regent to obtain CD3-positive T cells, and the time was recorded as the first day (D1). The cell medium used was the TexMACS GMP Medium (containing a 5% serum replacement), and the T cells were activated using the TransAct CD3/CD28 activating reagent (1:100) and IL-2 (200 IU/mL). After 24 hours of T cell activation, the cells were infected with the lentiviruses with different CARs prepared in Example 2 (MOI = 2). On day 7 (D7) after activation, an appropriate quantity of cells were taken from each group for CAR positive rate determination; medium replenishment was performed on D3 by adding the medium in a volume equal to the original volume. On D5 and D7, the cell density was adjusted to 5 × 10⁵ cells/mL, and the culture was continued. On D9, the cell killing and cytokine secretion level assays were performed.

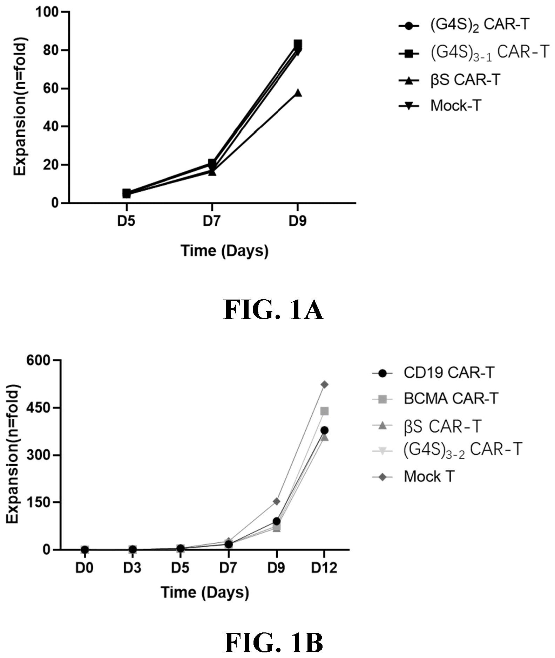

During the culture, the cell amplification folds are shown in FIGs. 1A and 1B. The results show that T cells expressing the CAR capable of simultaneously targeting CD19 and BCMA described in the present application can proliferate normally. The amplification folds of (G4S)ₙ CAR-T (n = 2 or 3) dual-target CAR-T at time points D5, D7, and D9 of culture were comparable to those of Mock-T and higher than those of βS CAR-T.

### Example 4. Determination of Positive Rate and Copy Number of CD19-BCMA Dual-Target CAR-T Cells

The CAR positive rate and CAR copy number per transduced cell are important indicators for evaluating the efficacy and safety. Due to the special structures of the dual-target CAR-Ts, there are cases where the CAR transduction efficiency is relatively low or the CAR copy number is excessively high, making such CARs fail to meet the criteria.

To evaluate the expression of the dual-target CARs, in this example, the CAR positive rate was determined by flow cytometry on day 7 after the activation of T cells from different donors. The determination results are shown in Table 6 below. The positive rates of the dual-target CAR-T groups were all above 30%, and the transduction efficiency of the CARs containing the G4S linker was higher than that of the CAR containing the βS linker.

**Table 6. Determination values of CAR positive rate of each group**

| Donor | Group | CAR positive rate (%) |
|---|---|---|
| Donor 1 | βS CAR-T | 33.3 |
| | (G4S)₂ CAR-T | 40.5 |
| | (G4S)₃₋₁ CAR-T | 39.0 |
| Donor 2 | βS CAR-T | 49.9 |
| | (G4S)₂ CAR-T | 55.9 |
| | (G4S)₃₋₁ CAR-T | 50.2 |

The copy number of the dual-target CAR-T was determined using the qPCR method. The brief determination steps were as follows:
1. The CAR-T cell suspension was taken, counted, and washed 1-5 times with PBS.
2. The supernatant was discarded, the DNase I reaction solution was added to the cell pellet to digest the residual exogenous plasmid DNA, and the resulting mixture was mixed well using a pipette and then placed in a water bath at 37 °C for 15 minutes.
3. The mixture was placed in a dry thermostat at 60 °C to completely inactivate the DNase I enzyme for 5 minutes, and the cell pellet was washed once with PBS.
4. Cellular DNA was extracted according to the steps described in the instructions of the Biomed's tissue/cell genomic DNA rapid extraction kit.
5. The PCR instrument was turned on, and a PCR reaction system was prepared. The PCR reaction program and the reaction plate were set up: A two-step reaction program was set up: predenaturation at 95 °C for 2 minutes; 40 cycles of 95 °C for 5 seconds and 60 °C for 15 seconds; the total reaction volume was 20 µL.
6. Start running and the data were collected for analysis.
7. CAR copy number calculation: Each cell genome contained 2 hALB genes, from which the CAR copy number of each CART-BCMA cell was calculated. The formula was as follows: CAR copy number = (2 × CAR determination value)/hALB determination value

The copy number may affect the function of the CAR-T cells. An excessively low copy number may result in insufficient *in vivo* persistence of CAR-T cells, thereby affecting the long-term efficacy. An excessively high copy number may result in excessive T cell activation. In addition, the higher the copy number, the greater the risk of secondary tumors caused by integration-related activation of oncogenes or inactivation of tumor suppressor genes. Determination of the copy number demonstrated that when different linkers were used to link the sequences recognizing CD19 and BCMA, differences in copy number existed. The CAR copy number of (G4S)ₙ CAR-T (n = 2 or 3) determined on D7 was lower than that of βS CAR-T. The dual-target CARs containing the G4S linker exhibited a structural advantage of a lower copy number under similar transduction efficiency.

**Table 7. Determination values of CAR copy number of each group**

| Donor | Group | CAR copy/transduced cell (D7) | |
|---|---|---|---|
| | | MOI=2 | MOI=1 |
| Donor 1 | CD19-BCMA (G4S)₂ CAR-T | 4.4 | / |
| | CD19-BCMA (G4S)₃₋₁ CAR-T | 3.4 | / |
| | CD19-BCMA βS CAR-T | 5.6 | / |
| Donor 2 | CD19-BCMA (G4S)₂ CAR-T | 8.7 | 7.2 |
| | CD19-BCMA (G4S)₃₋₁ CAR-T | 7.6 | 6.9 |
| | CD19-BCMA βS CAR-T | 10.7 | 9.6 |
| Donor 3 | CD19-BCMA (G4S)₂ CAR-T | 5.8 | 4.9 |
| | CD19-BCMA (G4S)₃₁ CAR-T | 4.9 | 3.5 |
| | CD19-BCMA βS CAR-T | 8.7 | 6.6 |

### Example 5. Assay on Killing of CD19-BCMA Dual-Target CAR-T Cells

In this example, the killing efficiency of the CD19-BCMA dual-target CAR-T cells described in the present application on tumor cells were determined. The T cells expressing the CD19-BCMA dual-target CAR described in the present application have a killing effect on tumor cells.

The dual-target CAR-T cells cultured to day 9 and the corresponding tumor cell lines (MM.1S: expressing BCMA but not expressing CD19; K562-CD19: expressing CD19 but not expressing BCMA; MM.1S-CD19: expressing BCMA and CD19; Daudi: expressing BCMA and CD19) were co-cultured, and the determination of the killing efficiency was performed by the method for lactate dehydrogenase (LDH) determination. Different effector-to-target ratios were set in the experiment, with target cells at 3 × 10⁴ cells/well. The total system was added to a 96-well round-bottom plate at 200 µL/well, with 3 replicate wells set for each group. After 16 h, the cells were lysed. After 1 h of lysis, the supernatant was collected by centrifugation, and the substrate was added for OD_{490 nm} determination. The killing percentage was calculated based on the determination results.

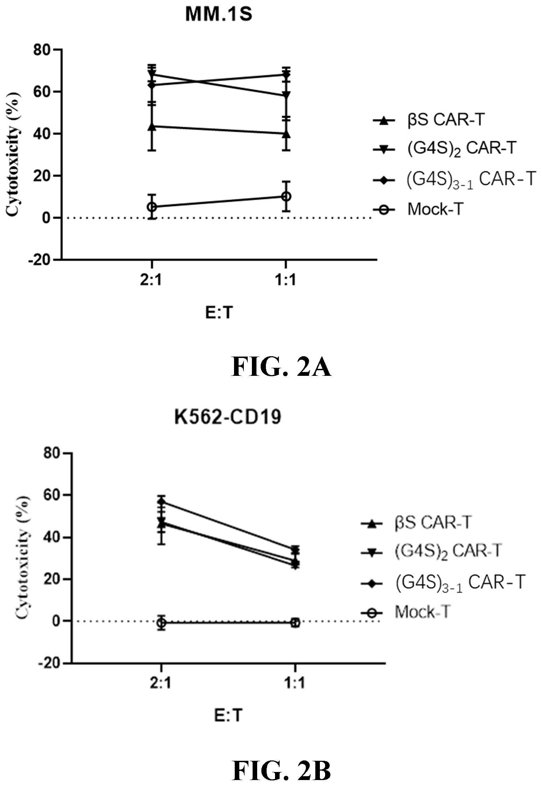

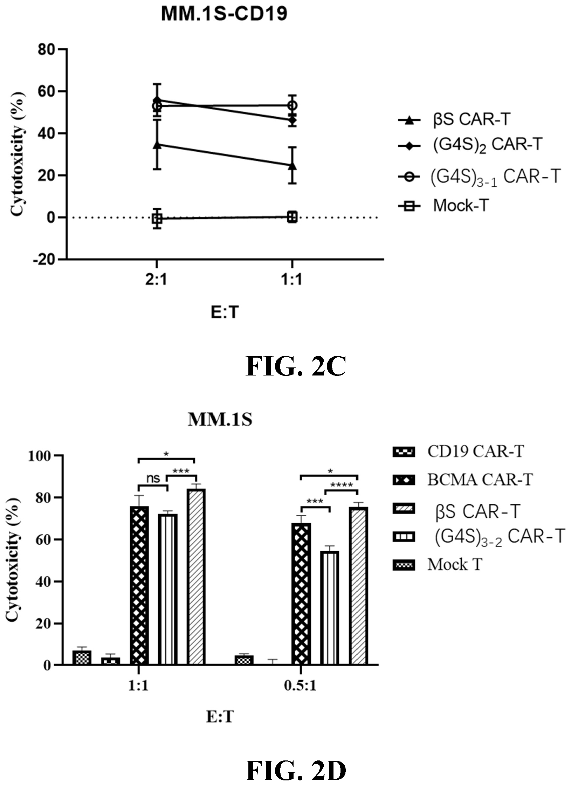

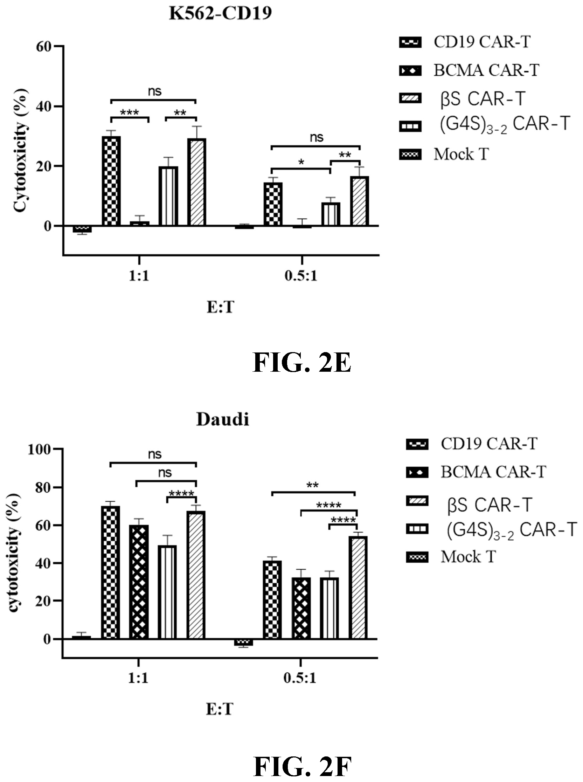

As shown in FIG. 2, the dual-target CAR-T cells described in the present application all exhibited killing effects on tumor cells. FIGs. 2A-2C compare the killing effects of CARs comprising different linkers, showing that in effector-to-target ratios of 2:1 and 1:1, the killing efficiency of (G4S)ₙ CAR-T (n = 2 or 3) on target cells MM.1S, K562-CD19, and MM.1S-CD19 was significantly improved as compared with that of βS CAR-T. FIGs. 2D-2F compare the killing effects of T cells expressing dual-target CARs and single-target CARs on different tumor cells. The dual-target CARs exhibited killing effects on tumor cells expressing BCMA, CD19, and BCMA and CD19. The results show that the relative positions of the CD19-targeting sequence and the BCMA-targeting sequence in the antigen-binding domain, as well as the selection of the linker, affected the killing effect of the dual-target CAR-T cells on tumor cells. By comprehensive comparison, the (G4S)₂ CAR-T and the (G4S)₃₋₁ CAR-T had stronger tumor cell killing effects.

### Example 6. Assay on Cytokine Secretion of CD19-BCMA Dual-Target CAR-Ts

In this example, it was determined that the CD19-BCMA dual-target CAR-T cells described in the present application can secrete cytokines after co-culture with tumor cells.

As described in Example 5, each group of T cells cultured on day 7 and each tumor target cell line were co-cultured for 16 hours, and then the cell killing was determined. In this example, an appropriate amount of the supernatant after 16 hours of co-culture at an effector-to-target ratio of 1:1 or 0.5:1 was collected for secretion determination of cytokines IFN-γ, TNF-α, IL-2, IL-6, and IL-10.

The steps for cytokine secretion determination can be found in the instructions of the Beadstar-mPlex^{®} mouse multi-cytokine assay kit.

The brief steps were as follows: The standard was diluted with deionized water according to the kit instructions. The supernatant samples were appropriately diluted with the solution C provided in the kit according to the estimated cytokine concentration. The solution A was fully vortexed for uniform dispersion, and the total required volume of the solution A and the solution B was calculated based on the sample amount, with 25 µL of the solution A and 25 µL of the solution B required for each sample. 25 µL of the cell supernatant sample, 25 µL of the serum sample, or 25 µL of each of the calibration samples at different concentrations was added to each well containing the mixed solution of the solution A and the solution B, and the mixture was gently and rapidly mixed thoroughly using a pipette without bubble generation. The tube was covered and incubated for 3 hours at room temperature with shaking at 200 rpm under light-shielded conditions. 900 µL of the washing solution was added to the incubated tube, and the mixture was mixed thoroughly by vortexing, followed by centrifugation at 2000 g for 2 minutes. The supernatant was carefully aspirated (brown dot sediment at the bottom represented magnetic fluorescent encoded microspheres). 100 µL of the washing solution (the solution E) was added, and the mixture was mixed thoroughly to prepare for the machine procedure. Sample data were collected by a flow cytometer, and data analysis was performed.

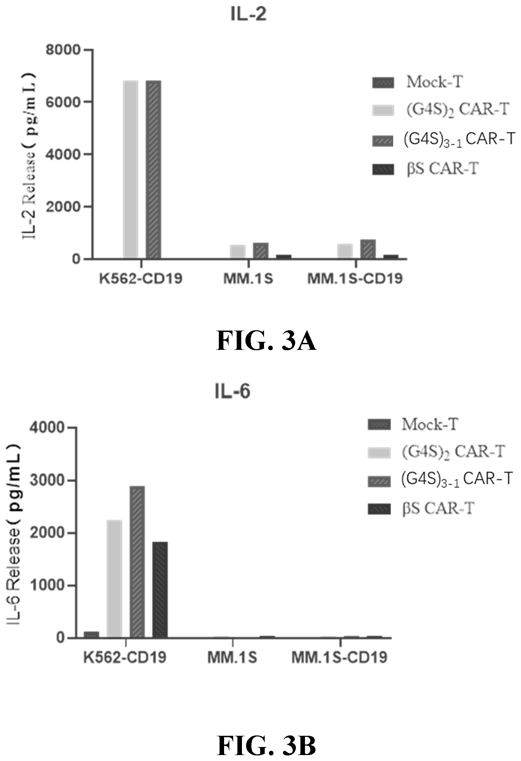

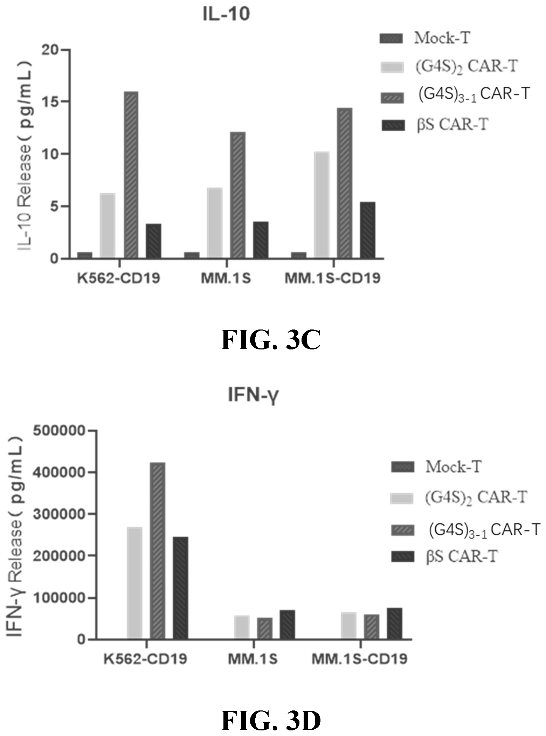

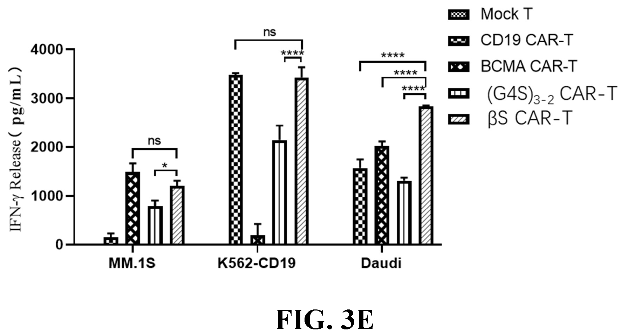

As shown in FIG. 3, the dual-target CAR-T cells described in the present application can secrete cytokines when co-cultured with tumor cells. FIGs. 3A-3D compare the cytokine secretion by CARs comprising different linkers. When co-cultured with different target cells, the dual-target CAR-T cells comprising different linkers can all secrete a certain amount of cytokines. Among them, the cytokine secretion level of (G4S)n CAR-T (n = 2 or 3) was higher than that of βS CAR-T. FIG. 3E compares the IFN-γ secretion levels after co-culturing T cells expressing dual-target CAR or single-target CAR with various tumor cell lines. When co-cultured with MM.1S expressing BCMA, the βS CAR-T group showed no significant difference in the IFN-γ secretion level compared with the single-target BCMA CAR-T group, yet its secretion level was significantly higher than that of the (G4S)₃₋₂ CAR-T group (P < 0.05). When co-cultured with K562-CD19 expressing a CD19 protein, the βS CAR-T group showed no significant difference in the IFN-γ secretion level compared with the single-target CD19 CAR-T group, but showed a significantly higher IFN-γ secretion level compared with the (G4S)₃₋₂ CAR-T group (P < 0.0001). When co-cultured with Daudi cells simultaneously expressing CD19 and BCMA proteins, the IFN-γ secretion level of the βS CAR-T group was significantly higher than that of all other groups (P < 0.0001). The results show that the relative positions of the CD19-targeting sequence and the BCMA-targeting sequence in the antigen-binding domain, as well as the selection of the linker, affected the cytokine secretion levels of dual-target CAR-T cells. Comprehensive comparison shows that the (G4S)₂ CAR-T and (G4S)₃₋₁ CAR-T exhibited significantly stronger cytokine secretion capabilities.

## Claims

1. A chimeric antigen receptor (CAR), comprising an extracellular antigen-binding domain that comprises a first antigen-binding domain and a second antigen-binding domain, wherein the first antigen-binding domain binds to CD19, and the second antigen-binding domain binds to BCMA; and:
1) the first antigen-binding domain comprises a first heavy chain variable region (VH) and a first light chain variable region (VL), wherein the first heavy chain variable region comprises the amino acid sequence set forth in SEQ ID NO: 3; and the first light chain variable region comprises the amino acid sequence set forth in SEQ ID NO: 1; and
2) the second antigen-binding domain comprises a second heavy chain variable region (VH), wherein the second heavy chain variable region comprises the amino acid sequence set forth in SEQ ID NO: 2.

2. The CAR according to claim 1, wherein the extracellular antigen-binding domain further comprises a linker linking the first heavy chain variable region, the first light chain variable region, and the second heavy chain variable region.

3. The CAR according to claim 2, wherein the linker comprises a β stranded linker and/or a G4S linker.

4. The CAR according to claim 3, wherein the G4S linker comprises a G4S3 linker and/or a G4S2 linker.

5. The CAR according to any one of claims 3-4, wherein the β stranded linker comprises an amino acid sequence set forth in SEQ ID NO: 6 or SEQ ID NO: 7.

6. The CAR according to any one of claims 4-5, wherein the G4S3 linker comprises the amino acid sequence set forth in SEQ ID NO: 4.

7. The CAR according to any one of claims 4-6, wherein the G4S2 linker comprises the amino acid sequence set forth in SEQ ID NO: 5.

8. The CAR according to any one of claims 1-7, wherein the extracellular antigen-binding domain comprises, from the N-terminus to the C-terminus:
1) the first heavy chain variable region, the linker, the first light chain variable region, the linker, and the second heavy chain variable region;
2) the first heavy chain variable region, the linker, the second heavy chain variable region, the linker, and the first light chain variable region;
3) the second heavy chain variable region, the linker, the first light chain variable region, the linker, and the first heavy chain variable region;
4) the second heavy chain variable region, the linker, the first heavy chain variable region, the linker, and the first light chain variable region;
5) the first light chain variable region, the linker, the second heavy chain variable region, the linker, and the first heavy chain variable region; or
6) the first light chain variable region, the linker, the first heavy chain variable region, the linker, and the second heavy chain variable region.

9. The CAR according to any one of claims 1-8, wherein the extracellular antigen-binding domain comprises, from the N-terminus to the C-terminus:
1) the first light chain variable region, the G4S2 linker, the second heavy chain variable region, the G4S2 linker, and the first heavy chain variable region;
2) the first light chain variable region, the G4S3 linker, the second heavy chain variable region, the G4S3 linker, and the first heavy chain variable region;
3) the first light chain variable region, the β stranded linker, the second heavy chain variable region, the β stranded linker, and the first heavy chain variable region; or
4) the second heavy chain variable region, the G4S3 linker, the first light chain variable region, the G4S3 linker, and the first heavy chain variable region.

10. The CAR according to any one of claims 1-9, wherein the extracellular antigen-binding domain comprises an amino acid sequence set forth in any one of SEQ ID NOs: 8-11.

11. The CAR according to any one of claims 1-10, further comprising a hinge region, a transmembrane domain, a co-stimulatory domain, and/or a signaling domain.

12. The CAR according to any one of claims 1-11, comprising, from the N-terminus to the C-terminus, the extracellular antigen-binding domain, the transmembrane domain, the hinge region, the co-stimulatory domain, and the signaling domain.

13. The CAR according to any one of claims 11-12, wherein the hinge region comprises a hinge region derived from one or more proteins in the following group: CD28, IgG1, IgG4, IgD, CD137, CD4, CD27, CD7, CD8, PD-1, ICOS, OX40, NKG2D, NKG2C, FcεRIγ, BTLA, GITR, DAP10, TIM1, SLAM, CD30, and LIGHT.

14. The CAR according to any one of claims 11-13, wherein the hinge region comprises a CD8 hinge region.

15. The CAR according to any one of claims 11-14, wherein the transmembrane domain comprises a transmembrane domain derived from one or more proteins in the following group: CD8, CD28, CD3e, CD3ε, CD137, CD4, CD27, CD7, PD-1, TRAC, TRBC, CD3ζ, CTLA-4, LAG-3, CD5, ICOS, OX40, NKG2D, 2B4, CD244, FcεRIγ, BTLA, CD30, GITR, HVEM, DAP10, CD2, NKG2C, LIGHT, DAP12, CD40L, CD154, TIM1, CD226, DR3, CD45, CD80, CD86, CD9, CD16, CD22, CD33, CD37, CD64, and SLAM.

16. The CAR according to any one of claims 11-15, wherein the transmembrane domain comprises a CD8 transmembrane domain.

17. The CAR according to any one of claims 11-16, wherein the co-stimulatory domain comprises a co-stimulatory domain derived from one or more proteins in the following group: CD28, 4-1BB, CD27, CD2, CD7, CD8A, CD8B, OX40, CD226, DR3, SLAM, CDS, ICAM-1, NKG2D, NKG2C, B7-H3, 2B4, FcεRIγ, BTLA, GITR, HVEM, DAP10, DAP12, CD30, CD40, CD40L, TIM1, PD-1, LFA-1, LIGHT, JAML, CD244, CD100, ICOS, CD40, and MyD88.

18. The CAR according to any one of claims 11-17, wherein the co-stimulatory domain comprises a 4-1BB co-stimulatory domain.

19. The CAR according to any one of claims 11-18, wherein the signaling domain comprises a signaling domain derived from a protein in the following group: CD3ζ, CD3δ, CD3γ, CD3ε, CD79a, CD79b, FceRIγ, FceRIβ, FcγRIIa, bovine leukemia virus gp30, Epstein-Barr virus (EBV) LMP2A, simian immunodeficiency virus PBj14 Nef, DAP10, DAP-12, and a domain protein comprising at least one ITAM.

20. The CAR according to any one of claims 11-19, wherein the signaling domain comprises a CD3ζ signaling domain.

21. The CAR according to any one of claims 1-20, comprising an amino acid sequence set forth in any one of SEQ ID NOs: 12-15.

22. An isolated nucleic acid molecule, wherein the isolated nucleic acid molecule encodes the CAR according to any one of claims 1-21.

23. The isolated nucleic acid molecule according to claim 22, comprising a nucleotide sequence set forth in any one of SEQ ID NOs: 16-19.

24. A vector, comprising the isolated nucleic acid molecule according to any one of claims 22-23.

25. The vector according to claim 24, comprising a viral vector.

26. The vector according to any one of claims 24-25, comprising a lentiviral vector.

27. A cell, wherein the cell comprises and/or expresses the CAR according to any one of claims 1-20, the isolated nucleic acid molecule according to any one of claims 22-23, and/or the vector according to any one of claims 24-26.

28. The cell according to claim 27, comprising an immune effector cell.

29. The cell according to any one of claims 27-28, comprising a T cell, a B cell, a natural killer cell (NK cell), a macrophage, an NKT cell, a γδ T cell, a monocyte, a dendritic cell, a granulocyte, a lymphocyte, a leukocyte, and/or a peripheral blood mononuclear cell.

30. The cell according to any one of claims 27-29, wherein the cell comprises a T cell.

31. The cell according to any one of claims 27-30, wherein the cell comprises a human cell.

32. A pharmaceutical composition, comprising the CAR according to any one of claims 1-21, the isolated nucleic acid molecule according to any one of claims 22-23, the vector according to any one of claims 24-26, and/or the cell according to any one of claims 27-31, and optionally a pharmaceutically acceptable carrier.

33. Use of the CAR according to any one of claims 1-21, the isolated nucleic acid molecule according to any one of claims 22-23, the vector according to any one of claims 24-26, and/or the cell according to any one of claims 27-31 in preparing a medicament for treating a disease or disorder associated with expression of CD19 and/or BCMA.

34. Use of the CAR according to any one of claims 1-21, the isolated nucleic acid molecule according to any one of claims 22-23, the vector according to any one of claims 24-26, the cell according to any one of claims 27-31, and/or the pharmaceutical composition according to claim 32 in treating a disease or disorder associated with expression of CD19 and/or BCMA.

35. A method for treating a disease or disorder associated with expression of CD19 and/or BCMA, comprising administering to a subject in need an effective amount of the CAR according to any one of claims 1-21, the isolated nucleic acid molecule according to any one of claims 22-23, the vector according to any one of claims 24-26, the immune effector cell according to any one of claims 27-31, and/or the pharmaceutical composition according to claim 32.

36. The use according to any one of claims 33-34 or the method according to claim 35, wherein the disease or disorder comprises a tumor, a hematological disease, and/or an autoimmune disease.

37. The use according to any one of claims 33-34 or the method according to claim 35, wherein the disease or disorder comprises a solid tumor and a non-solid tumor.

38. The use according to any one of claims 33-34 or the method according to claim 35, wherein the disease comprises lymphoma and/or leukemia.

39. The use according to any one of claims 33-34 or the method according to claim 35, wherein the disease comprises multiple myeloma, diffuse large B cell lymphoma, mantle cell lymphoma, small lymphocytic lymphoma, follicular lymphoma, acute myeloid leukemia, chronic myeloid leukemia, acute lymphocytic leukemia, chronic lymphocytic leukemia, non-Hodgkin's lymphoma, Hodgkin's lymphoma, megakaryocytic leukemia, Burkitt lymphoma, anaplastic large cell lymphoma, and/or mucosa-associated lymphoid tissue lymphoma.

40. The use according to any one of claims 33-34 or the method according to claim 35, wherein the disease comprises chronic Guillain-Barré syndrome, systemic lupus erythematosus, lupus nephritis, rheumatoid arthritis, Sjögren's syndrome, mixed connective tissue disease, multiple sclerosis, myasthenia gravis, demyelinating diseases, autoimmune hemolysis, idiopathic thrombocytopenic purpura, and/or idiopathic leukopenia.
